# EUROPEAN PATENT APPLICATION

(11) **EP 4 682 140 A1**
(43) Date of publication of application: **21.01.2026**
(21) Application number: 24189200.9
(22) Date of filing: 17.07.2024
(51) Int. Cl.: C07D 237/32

(54) **IMPROVED PROCESS FOR THE PREPARATION OF OLAPARIB**

(71) Applicant: F.I.S.- Fabbrica Italiana Sintetici S.p.A., 36075 Montecchio Maggiore (VI) (IT)
(72) Inventor: Semeraro, Floriana, 36075 Montecchio Maggiore (VI) (IT); Stabile, Paolo, 36075 Montecchio Maggiore (VI) (IT); Gesualdi, Fausto, 36075 Montecchio Maggiore (VI) (IT)

(57) **Abstract**

Object of the present invention is an improved process for the preparation of Olaparib of formula (9): 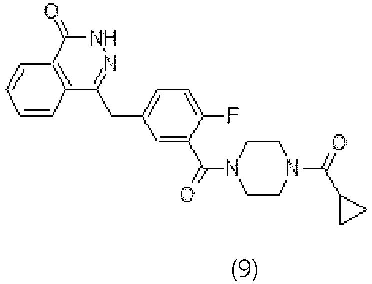 comprising the step of hydrolysis of intermediate of formula (11): 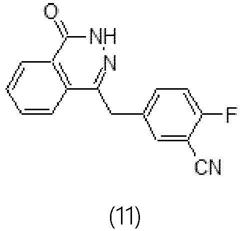 with a strong acid to give compound of formula (5): 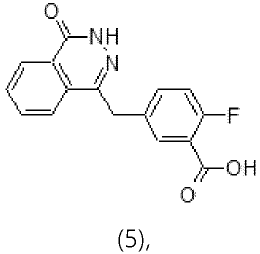 which is then converted to Olaparib of formula (9).

## Description

### Technical Field

The present invention refers to an improved process for the preparation of Olaparib.

### Background Art

Olaparib is a PARP inhibitor, inhibiting poly ADP ribose polymerase (PARP), indicated to treat breast cancer, ovarian cancer, fallopian tube cancer, peritoneal cancer, pancreatic cancer, and prostate cancer. In December 2014, Olaparib was approved for use as a single agent by the European Medicines Agency (EMA) in the European Union and by the Food and Drug Administration (FDA) in the United States.

Olaparib is a member of the class of N-acylpiperazines obtained by formal condensation of the carboxy group of 2-fluoro-5-[(4-oxo-3,4-dihydrophthalazin-1-yl)methyl]benzoic acid with the free amino group of N-(cyclopropylcarbonyl)piperazine.

Olaparib chemical name is 4-[[3-[4-(cyclopropanecarbonyl)piperazine-1-carbonyl]-4-fluorophenyl]methyl]-2H-phthalazin-1-one and Olaparib chemical structure corresponds to formula (9):

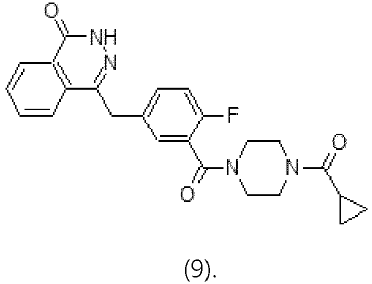

Olaparib can be prepared according to several synthetic processes described in the literature.

The first synthesis of Olaparib is described for example in WO 2004090876. The route of synthesis is attached in the following page. The synthesis starts with the convertion of hydroxyphthalide (1) into the dimethylphosphonate intermediate (2). By reaction of intermediate (2) with 2-fluoro-1-formylbenzonitrile (3), 2-fluoro-5-((3-oxoisobenzofuran-1(3H)-ylidene)methyl)benzonitrile (4) is obtained. Intermediate (4) is then reacted with aqueous sodium hydroxide and then with hydrazine, yielding 2-fluoro-5-((4-oxo-3,4-dihydrophthalazin-1-yl)methyl)benzoic acid (5). Olaparib (9) is finally obtained by reaction of intermediate (5) with 1-Boc-piperazine (6) followed by the reaction of the resulting amide (7) with cyclopropanecarbonyl chloride (8).

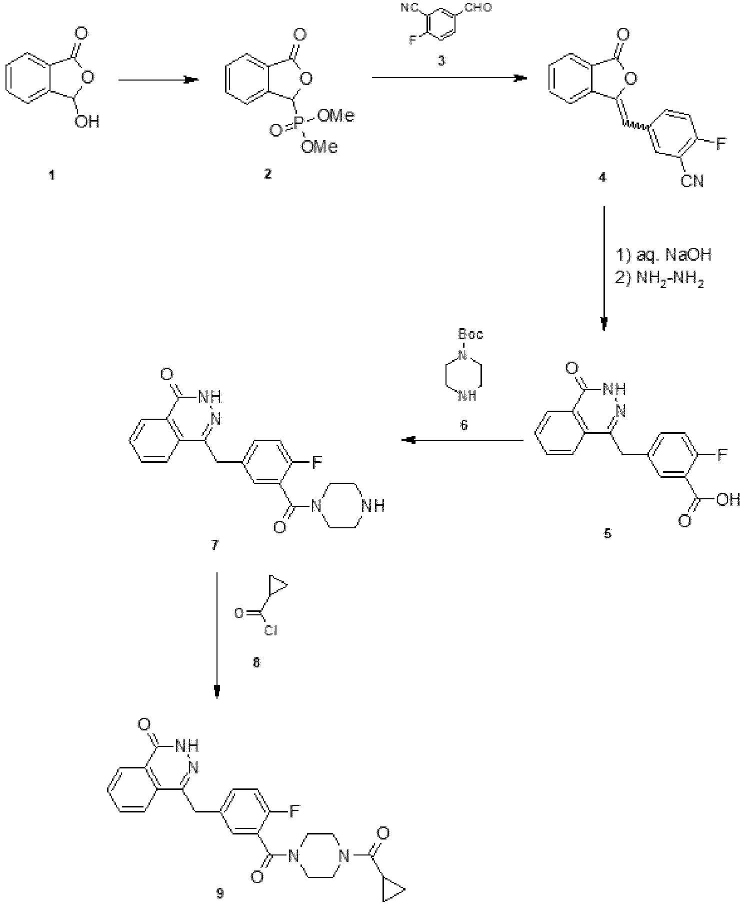

An improved route of synthesis towards Olaparib was later developed (WO2008047082), as depicted in the scheme attached in the following page. The synthesis starts with conversion of hydroxyphthalide (1) into diethylphosphonate (10). After reaction of intermediate (10) with 2-fluoro-1-formylbenzonitrile (3), 2-Fluoro-5-((3-oxoisobenzofuran-1(3H) - ylidene)methyl)benzonitrile (4) is obtained.

Intermediate (4) is then reacted with hydrazine and intermediate (11) is obtained. The nitrile group is hydrolized with sodium hydroxide, yielding 2-Fluoro-5-((4-oxo-3,4-dihydrophthalazin-1-yl)methyl)benzoic acid (5). Olaparib (9) is finally obtained by reaction of intermediate (5) with 1-Boc-piperazine (6) followed by the reaction of the resulting amide (7) with cyclopropanecarbonyl chloride (8).

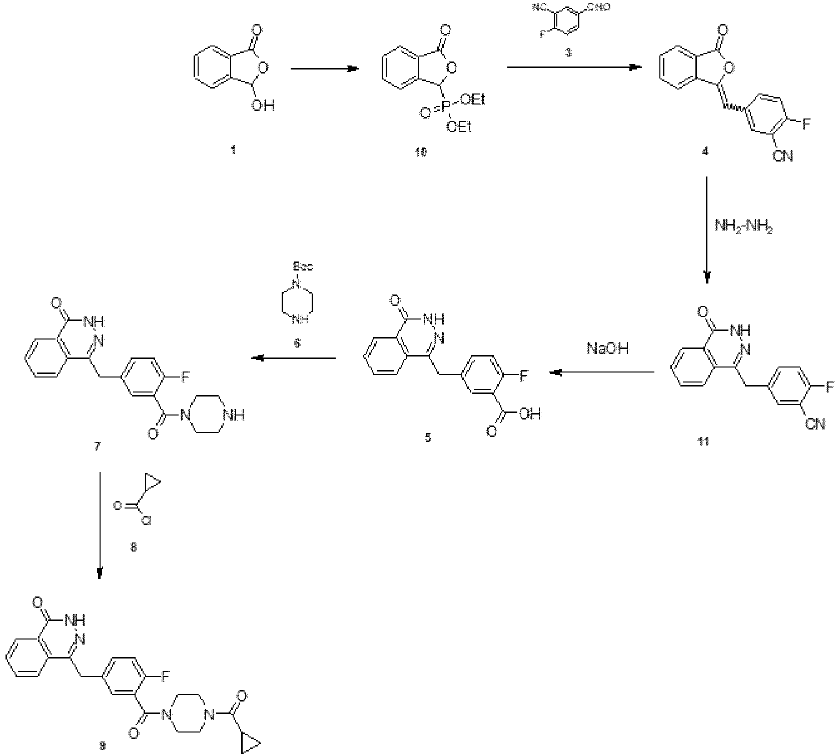

Both the methods are characterized by yields that are not up to the productivity standards desirable for modern API synthetic processes.

Furthermore, both the methods are unsatisafactory in controlling the purity of the process intermediates, which is a point of growing importance for the API manufacturing. In the pharmaceutical industry, in fact, it is well known that the purity of intermediates is essential in ensuring the efficacy and safety of the final drug product.

The development and production of intermediates for the APIs manufacturing, therefore, should tend towards high quality standards, as, for example, improved HPLC purity.

Thus, there is a need for a process for preparing Olaparib which is suitable for industrial manufacturing, with improved yield, process productivity and efficiency and characterized by higher quality of process intermediates.

### Summary of the invention

The problem addressed by the present invention is therefore that of providing a process for the preparation of Olaparib which is suitable for industrial manufacturing, characterized by improved yield, process productivity and efficiency and characterized by higher quality of process intermediates.

This problem is solved by a process for the preparation of Olaparib comprising the hydrolysis of key intermediate (11) under strong acidic conditions to obtain compound of formula (5), as reported in the scheme attached below.

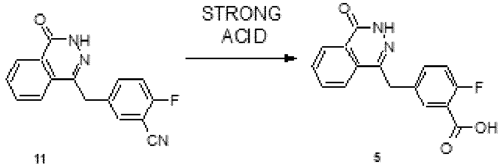

Specifically, the present invention provides a process for the preparation of Olaparib compound of formula (9): 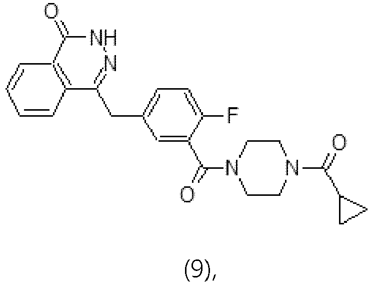 comprising the following steps:
a) hydrolysis reaction of compound of formula (11): 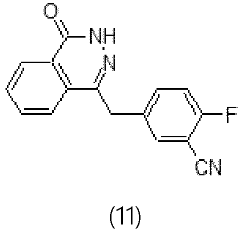 to give compound of formula (5): 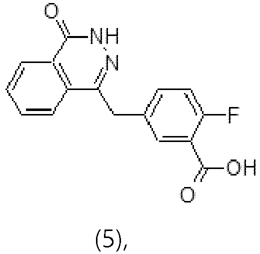 wherein the hydrolysis reaction is carried out in presence of a strong acid;
b) conversion of compound of formula (5) obtained in step a) to give Olaparib compound of formula (9):

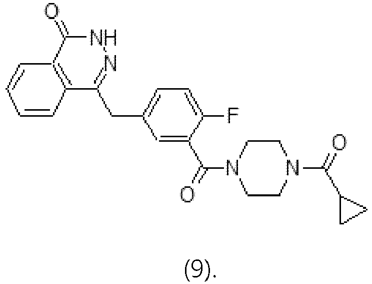

As a further aspect, the present invention provides a process for the preparation of compound of formula (5): 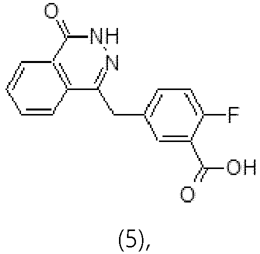 comprising hydrolysis reaction of compound of formula (11): 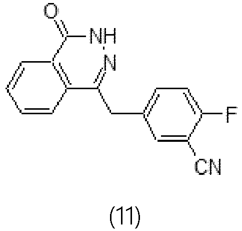 to give compound of formula (5): 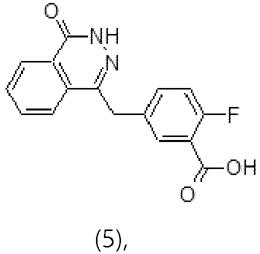 wherein the hydrolysis reaction is carried out in presence of a strong acid.

Further features and advantages of the processes according to the invention will result from the description hereafter reported of examples of realization of the invention, provided as an indication of the invention.

### Detailed description of the invention

The present invention is related to a process for the preparation of Olaparib compound of formula (9): 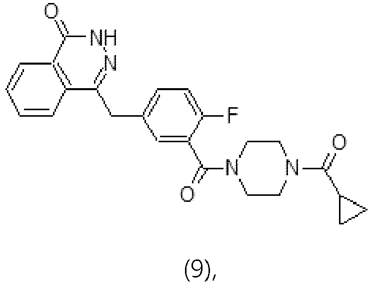 comprising the following steps:
a) hydrolysis reaction of compound of formula (11): 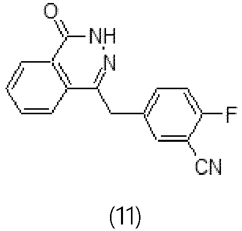 to give compound of formula (5): 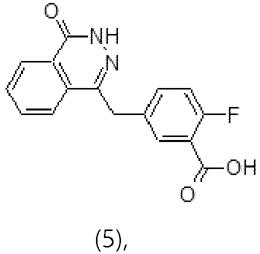 wherein the hydrolysis reaction is carried out in presence of a strong acid;
b) conversion of compound of formula (5) obtained in step a) to give Olaparib compound of formula (9):

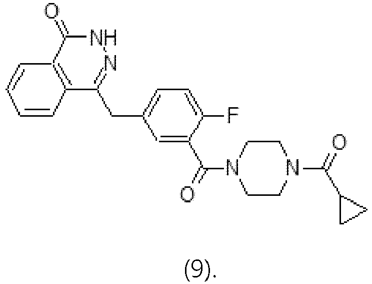

It was surprisingly found, in fact, that hydrolysis of the key intermediate of formula (11) with a strong acid according to the present invention allows to obtain key intermediate of formula (5) with considerably higher yields and HPLC purity with respect to prior art methods.

As already mentioned, in the route of synthesis for the preparation of Olaparib, key intermediate of formula (5) can be obtained for example by reacting compound of formula (4) with hydrazine and then hydrolising the nitrile function of compound of formula (11) thus obtained with sodium hydroxide to give compound of formula (5) with 77% overall molar yield and 96% HPLC purity (WO2004090876, example b).

Alternatively, key intermediate of formula (5) can be obtained by hydrolising the nitrile function of compound of formula (4) with sodium hydroxide and then reacting the hydrolised intermediate thus obtained with hydrazine to give compound of formula (5) with 84% overall molar yield (WO200804708, example 4, HPLC purity not reported).

It was surprisingly found, however, that by reacting compound of formula (4) with hydrazine and then hydrolysing compound of formula (11) thus obtained with a strong acid, as is the object of the present invention, compound of formula (5) can be obtained with 91.3% molar yield (average of the experimental results) starting from compound of formula (4), and with 99.2% HPLC purity (average of the experimental results).

### WO2004090876

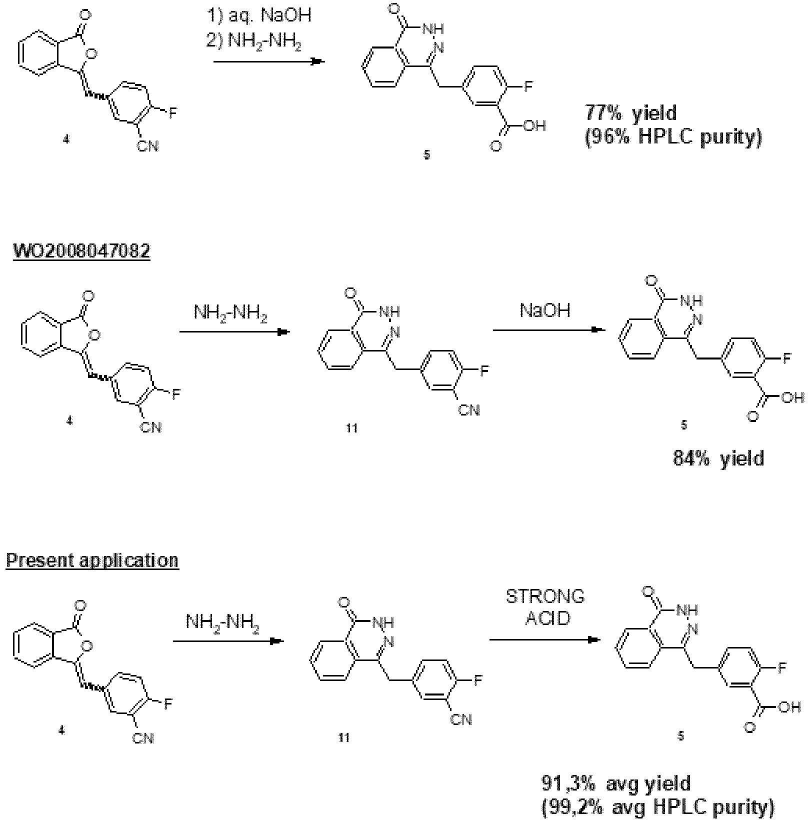

As exemplified in the scheme above, when the hydrolysis of the nitrile function is carried out under alkaline conditions, as in WO2004090876 and in WO200804708, the reaction with hydrazine can be performed both before and after said alkaline hydrolysis.

Conversely, it was surprisingly found that when the hydrolysis of the nitrile function is carried out under strong acidic conditions, as from the present application process, the reaction with hydrazine must be carried out before the nitrile function hydrolysis.

By carrying out the hydrolysis under strong acidic conditions on compound of formula (4), that is to say before reaction with hydrazine to generate the phtalazinone ring, as exemplified in the scheme attached below, in fact, compound of formula (14) is obtained instead of the expected compound of formula (13).

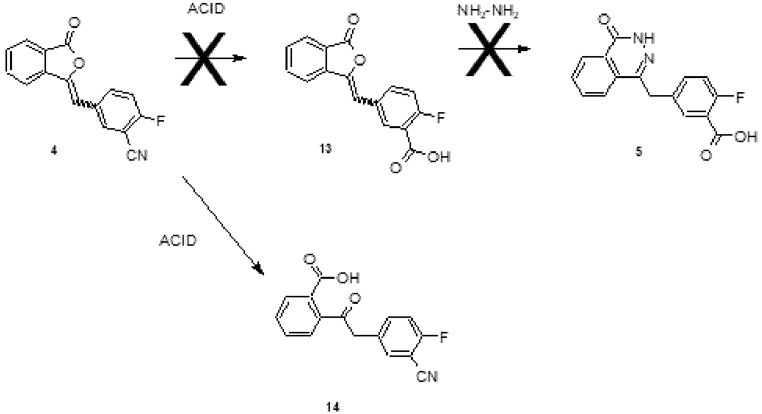

The present invention is therefore related to an improved process for the preparation of Olaparib compound of formula (9) comprising the hydrolysis reaction of compound of formula (11) with a strong acid to give compound of formula (5), followed by conversion of said compound of formula (5) to Olaparib (9).

It was surprisingly found that, among many possibilities for improving the process for the preparation of Olaparib (9), the acid hydrolysis of compound of formula (11) for providing compound of formula (5), provides excellent results in terms of yields, purity and overall process economy.

As shown in the scheme attached in the following page, conversion of compound of formula (5) to Olaparib (9) could comprise the reaction of compound (5) with compound (6) to give compound (7) followed by reaction of compound (7) with compound (8) to give Olaparib (9).

The synthetic procedure for the preparation of compound (7) by reaction of compound (5) with compound (6) is described, for example, in WO2004080976 (Example 1(b)).

The following reaction of compound (7) with compound (8) to give Olaparib (9) is described, for example, in WO2004080976 (Example 8, refer to the preparation of compound 152).

Alternatively, conversion of compound of formula (5) to Olaparib (9) could comprise the reaction of compound (5) with compound (12) to give Olaparib (9).

This reaction is described, for example, in US8247416 (Example 2).

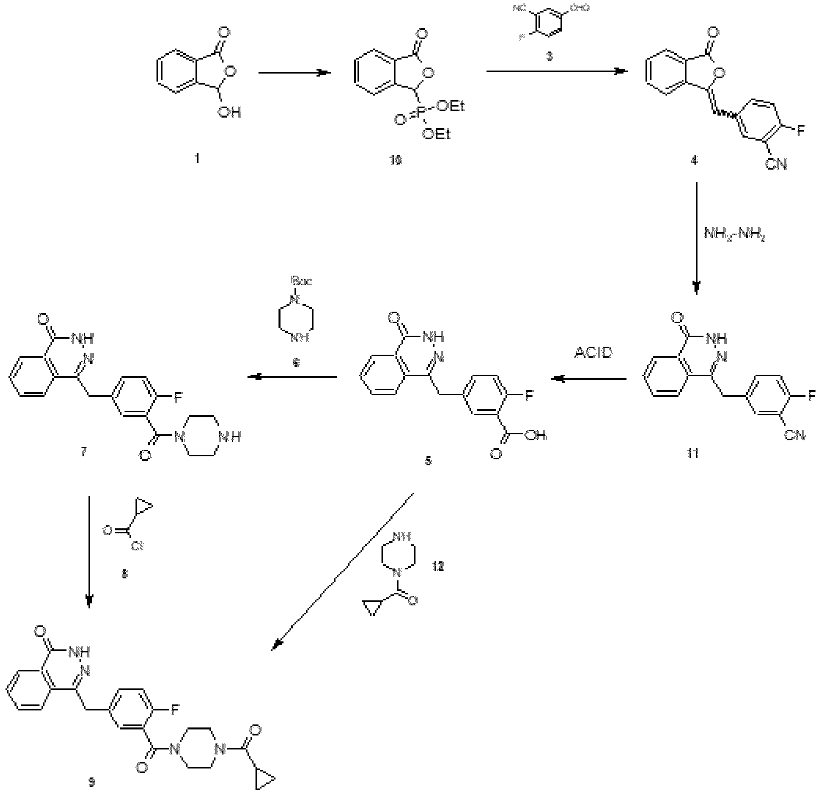

Therefore, according to a preferred embodiment, the present invention is related to a process for the preparation of Olaparib (9), wherein in step b) conversion of compound of formula (5) obtained in step a) to give Olaparib compound of formula (9), comprises either the following steps:
a1) reaction of compound of formula (5): 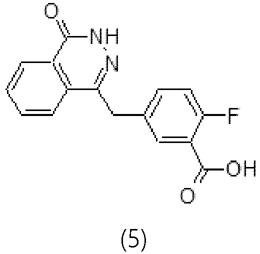 with compound of formula (6): 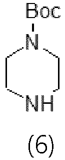 to give compound of formula (7): 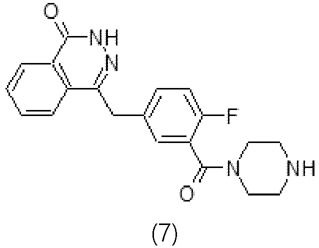
b1) reaction of compound of formula (7) obtained in step a1) with compound of formula (8): 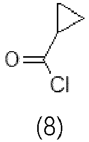 to give Olaparib of formula (9): 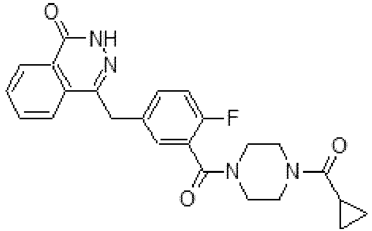 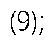 or the following step:
   a2) reaction of compound of formula (5): 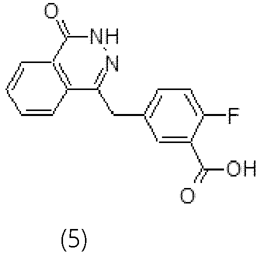 with compound of formula (12): 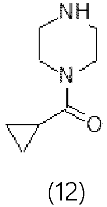 to give Olaparib of formula (9):

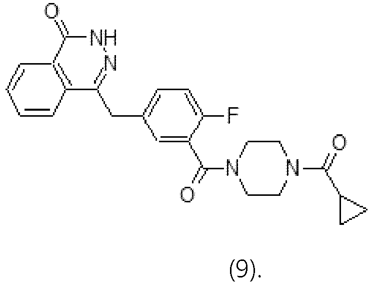

According to a preferred embodiment, the present invention is related to a process for the preparation of Olaparib (9) as reported above, wherein compound of formula (11) is obtained by reaction of compound of formula (4): 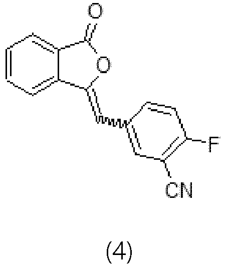 with hydrazine to give compound of formula (11):

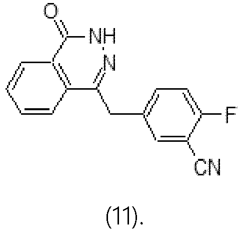

According to a preferred embodiment, the present invention is related to a process for the preparation of Olaparib (9) as reported above, wherein compound of formula (11) is not isolated.

According to a preferred embodiment, the present invention is also related to a process for the preparation of compound of formula (5): 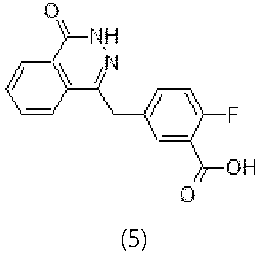 comprising hydrolysis reaction of compound of formula (11): 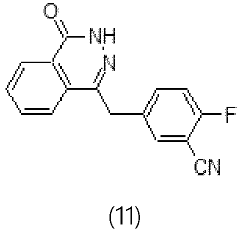 with a strong acid to give compound of formula (5).

In chemistry, a strong acid is an acid which ionizes (splits) completely in a solution of water. A strong acid always loses a proton when put in water. The acids commonly considered as strong acids in chemistry are hydrochloric acid, hydrobromic acid, hydroiodic acid, perchloric acid, nitric acid and sulfuric acid.

According to a preferred embodiment, in step a) the strong acid is selected from the list comprising hydrochloric acid, hydrobromic acid, hydroiodic acid, perchloric acid, nitric acid, or sulfuric acid.

According to a more preferred embodiment of the process of the present invention, in step a) the amount of acid is comprised within the range from 2.0 to 30.0 equivalents with respect to compound of formula (11).

Molar equivalent or equivalent (eq) means that the molar amount of a substance reacts with a molar amount of another substance in a given chemical reaction.

According to an even more preferred embodiment of the process of the present invention, in step a) the strong acid is sulfuric acid.

According to a preferred embodiment of the process of the present invention, in step a) the amount of sulfuric acid is comprised within the range from 2.0 to 15.0 equivalents or from 3.0 to 8.0 equivalents with respect to compound of formula (11).

According to a more preferred embodiment of the process of the present invention, in step a) a solvent selected from the group comprising acetic acid, chloroacetic acid, trifluoroacetic acid, formic acid, propionic acid is added within the range from 3.0 to 30.0 equivalents with respect to compound of formula (11).

According to a more preferred embodiment of the process of the present invention, in step a) the solvent selected is acetic acid.

According to a preferred embodiment of the process of the present invention, in step a) acetic acid is added as solvent within the range from 3.0 to 30.0 equivalents or from 8.0 to 15.0 equivalents with respect to compound of formula (11).

According to a more preferred embodiment of the process of the present invention, in step a) the strong acid is sulfuric acid in amount comprised within the range from 2.0 to 15.0 equivalents or from 3.0 to 8.0 equivalents and the solvent is acetic acid, in amount comprised within the range from 3.0 to 30.0 equivalents or from 8.0 to 15.0 equivalents with respect to compound of formula (11).

According to a preferred embodiment of the process of the present invention, in step a) the hydrolysis reaction is carried out at a temperature comprised in the range from 80°C to 120°C for a time comprised in the range from 6 hours to 30 hours.

According to a more preferred embodiment of the process of the present invention, in step a) the hydrolysis reaction is carried out at 100°C -120°C.

According to a more preferred embodiment of the process of the present invention, in step a) the hydrolysis reaction is carried out for 16 hours.

According to an even more preferred embodiment of the process of the present invention, in step a) the hydrolysis reaction is carried out at 100°C - 120°C for 16 hours.

According to a preferred embodiment, the present invention is also related to the use of a strong acid for the hydrolysis of compound of formula (11): 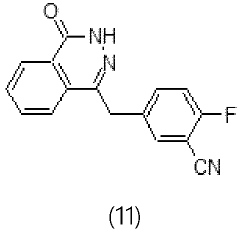 to give compound of formula (5):

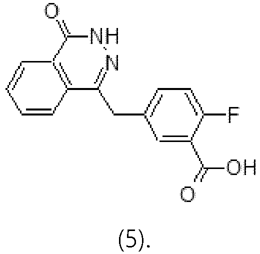

All the features and preferred embodiments of the process of the present invention given above can be combined in each possible combination to carry out the claimed process.

### Experimental Section

Compound (4) can be prepared, for example, according to Example b (synthesis of key intermediates) of WO2004090876 (page 46).

Compound (7) can be prepared starting from compound (5), for example, according to Example 1(b) of WO2004080976.

Olaparib (compound (9)) can be prepared, for example, starting from compound (7) according to WO2004080976 (Example 8, refer to the preparation of compound 152).

Room temperature (RT) means a temperature that is comprised in a range from 20 to 25°C, it is defined as comfortable temperature range indoors.

As intended herein, drying the wet material under *vacuum* means to heat the material to a given temperature for a given time while applying a reduced pressure. This operation is performed to remove residual solvents from the product.

As intended herein, to concentrate a mixture under *vacuum* means to heat the material to a given temperature for a given time while applying a reduced pressure. This operation is performed to remove solvents from the mixture.

### Example 1: Preparation of compound of formula (5).

In a round bottom flask equipped with a half-moon stirrer were charged at RT: 50 g of compound of formula (4), 250 mL of purified water and 50 mL of isopropanol. Thereafter, 22.9 mL of hydrazine hydrate were dosed in 20 minutes over the resulting suspension maintaining the same temperature. The mixture was then heated to 85°C and kept stirring at this temperature for 7.5 hours. After concentration to residue under *vacuum* keeping the internal temperature within the range 55-60°C, 300 ml of purified water were added. This operation was repeated until unreacted hydrazine was reduced below 1000 ppm (5 times). The mixture was diluted at 55-60°C with 250 mL of acetic acid and 150 mL of 98% sulfuric acid were then added to the mixture in 40 minutes. The resulting mixture was heated to 100-115°C for 23 hours, then 150 mL of purified water were added in 30 minutes. After cooling down the mixture to T=45°C over 1 hour, the suspension was kept stirring at this temperature for 2 hours and then filtered, washing the cake three times with 150 mL of purified water and finally with 100 mL of isopropanol. The wet solid was then dried under *vacuum* at T=60°C for 11 hours.

50.5 g of compound of formula (5) were obtained (89.8% molar yield, 99.6% A/A% by HPLC).

### Example 2: Preparation of compound of formula (5).

In a round bottom flask equipped with a half-moon stirrer were charged at RT: 50 g of compound of formula (4), 250 mL of purified water and 50 mL of isopropanol. Thereafter, 28.6 mL of hydrazine hydrate were dosed in 20 minutes over the resulting suspension maintaining the same temperature. The mixture was then heated to 85°C and kept stirring at this temperature for 2.5 hours. After concentration to residue under *vacuum* keeping the internal temperature within the range 55-60°C, 300 ml of purified water were added. This operation was repeated until unreacted hydrazine was reduced below 1000 ppm (6 times). The mixture was diluted at 55-60°C with 150 mL of purified water and 75 mL of acetic acid, then 150 mL of 98% sulfuric acid were then added to the mixture in 30 minutes. The resulting mixture was heated to 100-115°C for 14.5 hours, then 150 mL of purified water were added in 30 minutes. After cooling down the mixture to T=43°C over 1 hour, the suspension was kept stirring at this temperature for 4.5 hours and then filtered, washing the cake three times with 150 mL of purified water and finally with 100 mL of isopropanol. The wet solid was then dried under *vacuum* at T=60°C for 12.5 hours.

51.1 g of compound of formula (5) were obtained (90.9% molar yield, 98.7% A/A% by HPLC).

### Example 3: Preparation of compound of formula (5).

In a round bottom flask equipped with a half-moon stirrer were charged at RT: 100 g of compound of formula (4), 500 mL of purified water and 100 mL of isopropanol. Thereafter, 57.3mL of hydrazine hydrate were dosed in 20 minutes over the resulting suspension maintaining the same temperature. The mixture was then heated to 85°C and kept stirring at this temperature for 6 hours. After concentration to residue under *vacuum* keeping the internal temperature within the range 55-60°C, 300 ml of purified water were added. This operation was repeated until unreacted hydrazine was reduced below 1000 ppm (10 times). The mixture was diluted at 55-60°C with 150 mL of acetic acid, then 300 mL of 98% sulfuric acid were then added to the mixture in 30 minutes. The resulting mixture was heated to 98-100°C for 17 hours, then 300 mL of purified water were added in 30 minutes. After cooling down the mixture to T=43-45°C over 1.5 hours, the suspension was kept stirring at this temperature for 3.5 hours and then filtered, washing the cake three times with 300 mL of purified water and finally with 200 mL of isopropanol. The wet solid was then dried under *vacuum* at T=60°C for 14 hours.

102.7g of compound of formula (5) were obtained (91.3% molar yield, 98.1% A/A% by HPLC).

### Example 4: Preparation of compound of formula (11).

In a round bottom flask equipped with a half-moon stirrer were charged at RT 100 g of compound of formula (4) and 600 mL of isopropanol. Thereafter, 34.4mL of hydrazine hydrate were dosed in 20 minutes over the resulting suspension maintaining the same temperature. The mixture was then heated to 80°C and kept stirring at this temperature for 3.5 hours. After removal of 201 mL of solvent via a Dean-Stark apparatus the mixture is cooled to 20°C and 300 mL of purified water are added. Maintaining the same temperature, 60 mL of acetic acid were dosed in 30 minutes, and the resulting mixture was kept stirring at this temperature for further 3 hours. The suspension was filtered, washing the cake two times with 200 mL of purified water and finally with 100 mL of isopropanol. The wet solid was then dried under *vacuum* at T=60°C for 11 hours. 102.2g of compound (11) were obtained.

### Example 5: Preparation of compound of formula (5).

In a round bottom flask equipped with a half-moon stirrer were charged at RT 100 g of compound of formula (11) obtained in Example 4, 300 mL of purified water and 150 mL of acetic acid. The mixture was heated to T=40°C, then 300 mL of 98% sulfuric acid were added in 30 minutes. The resulting mixture was heated to 100°C for 16 hours, then it was cooled to 45°C over 2 hours and 300 mL of purified water were added in 25 minutes. The mixture was stirred at T=42-47°C for 1 hour and the suspension was filtered, washing the cake two times with 300 mL of purified water and finally with 200 mL of isopropanol. The wet solid was then dried under *vacuum* at T=60°C for 11 hours.

104.6g of compound of formula (5) were obtained (95.0% overall molar yield of Example 4 and Example 5, 98.9% A/A% by HPLC).

### Example 6: Preparation of compound of formula (11).

In a round bottom flask equipped with a half-moon stirrer were charged at RT 100 g of compound of formula (4) and 600 mL of isopropanol. Thereafter, 34.4mL of hydrazine hydrate were dosed in 20 minutes over the resulting suspension maintaining the same temperature. The mixture was then heated to 82°C and kept stirring at this temperature for 3 hours. After removal of 200 mL of solvent via a Dean-Stark apparatus the mixture is cooled to RT and 300 mL of purified water are added. Maintaining the same temperature, the pH of the mixture was adjusted to pH=5 by adding 60 mL of acetic acid, and the resulting mixture was kept stirring at this temperature for further 2 hours. The suspension was filtered, washing the cake two times with 200 mL of purified water and finally with 100 mL of isopropanol. The wet solid was then dried under *vacuum* at T=60°C for 11 hours. 102.3g of compound (11) were obtained.

### Example 7: Preparation of compound of formula (5).

In a round bottom flask equipped with a half-moon stirrer were charged at RT 100 g of compound of formula (11) obtained in Example 6, 300 mL of purified water and 150 mL of acetic acid. The mixture was heated to T=45°C, then it was cooled to T=65°C and 300 mL of 98% sulfuric acid were added in 1 hour. The resulting mixture was heated to 100°C for 16 hours, then 300 mL of purified water were added in 30 minutes. After cooling down the mixture to T=45°C for 1 hour the suspension was filtered, washing the cake two times with 300 mL of purified water and finally with 200 mL of isopropanol. The wet solid was then dried under *vacuum* at T=60°C for 21 hours.

103.6g of compound of formula (5) were obtained (94.1% overall molar yield of Example 6 and Example 7, 99.5% A/A% by HPLC).

### Example 8: Preparation of compound of formula (11).

In a round bottom flask equipped with a half-moon stirrer were charged at RT 20 g of compound of formula (4) and 120 mL of isopropanol. Thereafter, 11.5 mL of hydrazine hydrate were dosed in 15 minutes over the resulting suspension maintaining the same temperature. The mixture was then heated to 80°C and kept stirring at this temperature for 3 hours. After removal of 40 mL of solvent via a Dean-Stark apparatus the mixture is cooled to RT and 60 mL of purified water are added. Maintaining the same temperature, the pH of the mixture was adjusted to pH=4.5-5 by adding 19 mL of acetic acid, and the resulting mixture was kept stirring at this temperature for further 1 hour. The suspension was filtered, washing the cake two times with 40 mL of purified water. The wet solid was then dried under *vacuum* at T=60°C for 12 hours. 20.3g of compound (11) were obtained.

### Example 9: Preparation of compound of formula (5).

In a round bottom flask equipped with a half-moon stirrer were charged at RT 10 g of compound of formula (11) obtained in Example 8, 30 mL of purified water and 15 mL of acetic acid. The mixture was heated to T=50°C, then 10 mL of 98% sulfuric acid were added in 1 hour. The resulting mixture was heated to 100°C and 15 mL of acetic acid were added. Further 10 mL of 98% sulfuric acid were added thereafter and the mixture was kept stirring at T=117°C for 25 hours. After cooling the mixture to T=70-60°C, 30 mL of purified water were added in 15 minutes. After cooling down the mixture to T=45°C for 1 hour the suspension was filtered, washing the cake two times with 30 mL of purified water and finally with 20 mL of isopropanol. The wet solid was then dried under *vacuum* at T=60°C for 13.5 hours.

9.9g of compound of formula (5) were obtained (89.4% overall molar yield of Example 8 and Example 9, 99.6% A/A% by HPLC).

### Example 10: Preparation of compound of formula (11).

In a round bottom flask equipped with a half-moon stirrer were charged at RT 20 g of compound of formula (4) and 120 mL of isopropanol. Thereafter, 4 mL of hydrazine hydrate were dosed in 10 minutes over the resulting suspension maintaining the same temperature. The mixture was then heated to 80°C and kept stirring at this temperature for overall 6 hours. During the stirring at T=80°C, further 2.9 mL of hydrated hydrazine were added. After removal of 41 mL of solvent via a Dean-Stark apparatus, the mixture is cooled to RT and 60 mL of purified water are added. Maintaining the same temperature, 12 mL of acetic acid were added, and the resulting mixture was kept stirring at this temperature for further 1 hour 15 minutes. The suspension was filtered, washing the cake two times with 40 mL of purified water. The wet solid was then dried under *vacuum* at T=60°C for 11 hours. 20.5 g of compound (11) were obtained.

### Example 11: Preparation of compound of formula (5).

In a round bottom flask equipped with a half-moon stirrer were charged at RT 10 g of compound of formula (11) obtained in Example 10, 30 mL of purified water and 15 mL of acetic acid. The mixture was heated to T=44-47°C, then 10 mL of 98% sulfuric acid were added in 1 hour. The resulting mixture was heated to 116°C for 28 hours. After cooling the mixture to T=70-60°C, 30 mL of purified water were added in 1 hour. After cooling down the mixture to T=45°C for 1 hour the suspension was filtered, washing the cake two times with 30 mL of purified water and finally with 20 mL of isopropanol. The wet solid was then dried under *vacuum* at T=60°C for 23 hours. 10 g of compound of formula (5) were obtained (91.1% overall molar yield of Example 10 and Example 11, 99.8% A/A% by HPLC).

### Example 12: Preparation of compound of formula (11).

In a round bottom flask equipped with a half-moon stirrer were charged at RT 20 g of compound of formula (4) and 120 mL of isopropanol. Thereafter, 2 mL of hydrazine hydrate were dosed in 5 minutes over the resulting suspension maintaining the same temperature. The mixture was then heated to 80°C and kept stirring at this temperature for 4.5 hours. The suspension was cooled to 70 °C and 2 mL of hydrazine hydrate were added, then the mixture was heated to 80 °C and stirred for 3.5 hours. The suspension was cooled again to 70 °C and 2.3 mL of hydrazine hydrate were dosed, then the mixture was heated to 80 °C and stirred for 2 hours. After removal of 40 mL of solvent via a Dean-Stark apparatus, the mixture is cooled to RT and 60 mL of purified water are added. Maintaining the same temperature, the pH was adjusted to pH=4.5-5 with 9.5 mL of acetic acid, and the resulting mixture was kept stirring at this temperature for 1 hour. The suspension was filtered, washing the cake two times with 40 mL of purified water. The wet solid was then dried under *vacuum* at T=60°C for 11 hours. 20.4g of compound (11) were obtained.

### Example 13: Preparation of compound of formula (5).

In a round bottom flask equipped with a half-moon stirrer were charged at RT 10 g of compound of formula (11) obtained in Example 12,10 mL of purified water and 20 mL of acetic acid. The mixture was heated to T=45°C, then 10 mL of 98% sulfuric acid were added in 1 hour. The resulting mixture was heated to 120°C for 7 hours. After cooling the mixture to T=55°C, 30 mL of purified water were added in 30 minutes. After cooling down the mixture to T=45°C for 1 hour the suspension was filtered, washing the cake two times with 20 mL of purified water and finally with 10 mL of isopropanol. The wet solid was then dried under *vacuum* at T=60°C for 23 hours.

9.8g of compound of formula (5) were obtained (88.9% overall molar yield of Example 12 and Example 13, 99.0% A/A% by HPLC).

### Example 14: Preparation of compound of formula (5).

In a round bottom flask equipped with a half-moon stirrer were charged at RT 10 g of compound of formula (11) obtained in Example 12,15 mL of purified water and 30 mL of acetic acid. The mixture was heated to T=45°C, then 15 mL of 98% sulfuric acid were added in 1.5 hours. The resulting mixture was heated to 120°C for 8 hours. After cooling the mixture to T=55°C, 30 mL of purified water were added in 30 minutes. After cooling down the mixture to T=45°C for 3 hours the suspension was filtered, washing the cake two times with 20 mL of purified water, the with 10 mL of isopropanol and finally with 20 mL of isopropanol. The wet solid was then dried under *vacuum* at T=60°C for 14.5 hours.

10.1g of compound of formula (5) were obtained (91.6% overall molar yield of Example 12 and Example 14, 99.1% A/A% by HPLC).

### Example 15: Comparison between prior art and present application process performances.

In the table below are collected the molar yield and HPLC purity of compound of formula (5) obtained starting from compound of formula (4) following prior art processes reported in WO2004080976 and in WO2008047082 and following the present application process. The entry at the bottom of the table represents the average molar yield and the average HPLC purity of the process of the present application.

| **Experiment Molar yield HPLC purity Prior art methods** | | |
|---|---|---|
| WO2004080976 Exp b (nitrile interm. NOT isolated) | **77%** | **96%** |
| WO2008047082 Exp 4 (nitrile interm. isolated) | **84%** | not reported |

| **Method of invention** | | |
|---|---|---|
| EXAMPLE 1 (nitrile interm. NOT isolated) | 89.8% | 99.6% |
| EXAMPLE 2 (nitrile interm. NOT isolated) | 90.9% | 98.7% |
| EXAMPLE 3 (nitrile interm. NOT isolated) | 91.3% | 98.1% |
| EXAMPLE 4 and EXAMPLE 5 (nitrile interm. isolated) | 95.0% | 98.9% |
| EXAMPLE 6 and EXAMPLE 7 (nitrile interm. isolated) | 94.1% | 99.5% |
| EXAMPLE 8 and EXAMPLE 9 (nitrile interm. isolated) | 89.4% | 99.6% |
| EXAMPLE 10 and EXAMPLE 11 (nitrile interm. isolated) | 91.1% | 99.8% |
| EXAMPLE 12 and EXAMPLE 13 (nitrile interm. isolated) | 88.9% | 99.0% |
| EXAMPLE 12 and EXAMPLE 14 (nitrile interm. isolated) | 91.6% | 99.1% |
| **Average Method of invention** | **91.3%** | **99.2%** |

The results reported in the table clearly show that the present application process allows to obtain key intermediate of formula (5) with a considerably higher yield and/or HPLC purity with respect to the processes known in the prior art.

### Example 16: Analytical method for determining the chemical purity of the compounds in examples via HPLC:

- Waters X-Select CSH C18, 150 x 4.6 mm, 3.5µm, or equivalent;
- Temp. Column: 35°C;
- Mobile Phase A: Milli-Q Water + 0.1% v/v perchloric acid;
- Mobile Phase B: Acetonitrile + 0.1% v/v perchloric acid;
- Gradient

| Time (min) | %A | %B |
|---|---|---|
| 0 | 90 | 10 |
| 15 | 10 | 90 |
| 17 | 10 | 90 |
| 17.1 | 90 | 10 |
| 25 | 90 | 10 |

- Flow: 1.0 mL/min;
- UV Detector: 280 nm;
- Injection Volume: 5 µL;
- Analysis Time: 17 min;
- Post-run Time: 8 min;
- Diluent: Milli-Q Water/THF 50/50 v/v + 0.1% v/v perchloric acid.

## Claims

1. Process for the preparation of Olaparib compound of formula (9): 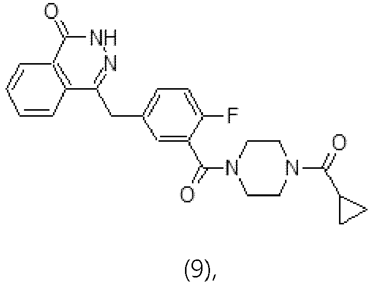 comprising the following steps:
a) hydrolysis reaction of compound of formula (11): 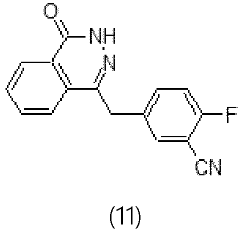 to give compound of formula (5): 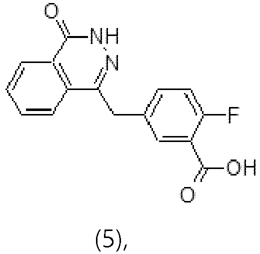 wherein the hydrolysis reaction is carried out in presence of a strong acid;
b) conversion of compound of formula (5) obtained in step a) to give Olaparib compound of formula (9):

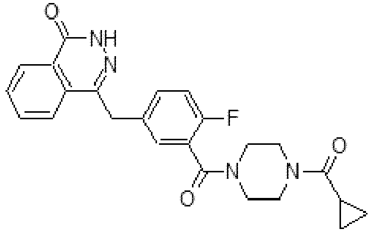

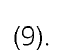

2. Process according to claim 1, wherein in step b) conversion of compound of formula (5) obtained in step a) to give Olaparib compound of formula (9) comprises either the following steps:
a1) reaction of compound of formula (5): 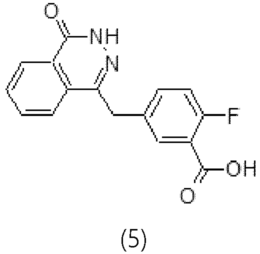 with compound of formula (6): 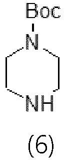 to give compound of formula (7): 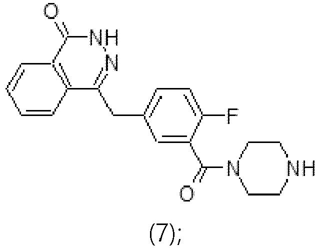
b1) reaction of compound of formula (7) obtained in step a1) with compound of formula (8): 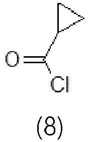 to give Olaparib of formula (9): 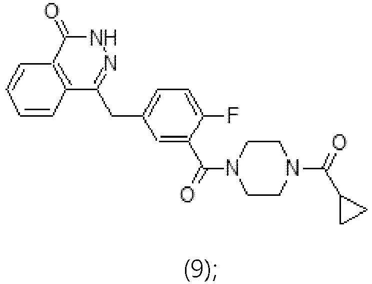 or the following step:
a2) reaction of compound of formula (5): 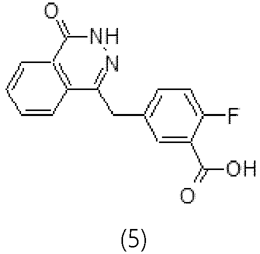 with compound of formula (12): 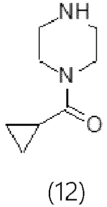 to give Olaparib of formula (9):

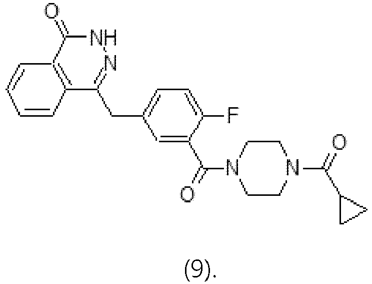

3. Process according to anyone of the claims from 1 to 2, wherein compound of formula (11) is obtained by reaction of compound of formula (4): 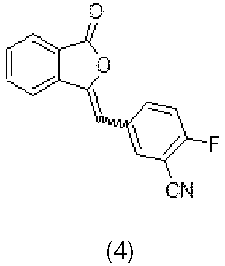 with hydrazine, to give compound of formula (11).

4. Process according to claim 3, wherein compound of formula (11) is not isolated.

5. Process according to any one of the claims 1 or 3 or 4, wherein in step a) the strong acid is selected from the list comprising hydrochloric acid, hydrobromic acid, hydroiodic acid, perchloric acid, nitric acid, or sulfuric acid.

6. Process according to any one of the claims from 1 to 5, wherein in step a) the amount of strong acid is comprised within the range from 2.0 to 30.0 molar equivalents with respect to compound of formula (11).

7. Process according to any one of the claims from 1 to 6, wherein in step a) the selected acid is sulfuric acid.

8. Process according to claim 7, wherein in step a) the amount of sulfuric acid is comprised within the range from 2.0 to 15.0 molar equivalents or from 3.0 to 8.0 molar equivalents with respect to compound of formula (11).

9. Process according to any one of claims from 1 to 8, wherein in step a) a solvent selected from the group comprising acetic acid, chloroacetic acid, trifluoroacetic acid, formic acid, propionic acid is added within the range from 3.0 to 30.0 molar equivalents with respect to compound of formula (11).

10. Process according to any one of the claims from 1 to 8, wherein in step a) acetic acid is added as solvent within the range from 3.0 to 30.0 molar equivalents or from 8.0 to 15.0 molar equivalents with respect to compound of formula (11).

11. Process according to any one of the claims from 1 to 10, wherein in step a) the hydrolysis reaction is carried out at a temperature comprised in the range from 80°C to 120°C for a time comprised in the range from 6 hours to 30 hours.

12. Process according to any one of the claims from 1 to 11, wherein in step a) the hydrolysis reaction is carried out at 110-120°C for 16 hours.

13. Process for the preparation of compound of formula (5): 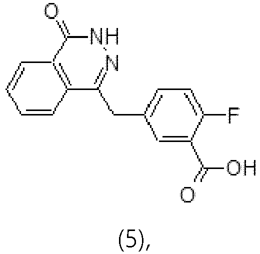 comprising hydrolysis reaction of compound of formula (11): 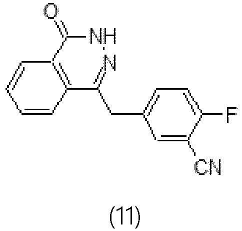 to give compound of formula (5): 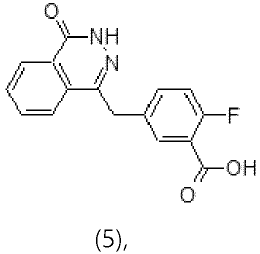 wherein the hydrolysis reaction is carried out in presence of a strong acid.

14. Process according to claim 13, wherein the reaction is carried out according to any one of the claims from 3 to 12.

15. Use of a strong acid for the hydrolysis of compound of formula (11): 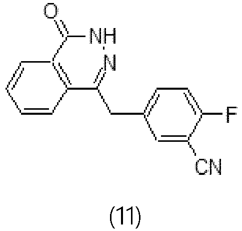 to give compound of formula (5):
